# EUROPEAN PATENT SPECIFICATION

(11) **EP 0 357 377 B1**
(45) Date of publication and mention of the grant of the patent: **19.10.1994**
(21) Application number: 89308728.8
(22) Date of filing: 30.08.1989
(51) Int. Cl.: A61B 17/12, B25B 5/12

(54) **Toggle action aneurysm clip**
Kniehebelklammer für Pulsadergeschwulst
Pince à levier articulé pour anévrisme

(30) Priority: 31.08.1988 US 238666
(43) Date of publication of application: 07.03.1990
(73) Proprietor: CODMAN & SHURTLEFF INC., Randolph Massachusetts 02368 (US)
(72) Inventor: Kees, George, Jr., Wilder Kentucky 41071 (US)
(74) Representative: Fisher, Adrian John

(56) References cited:
- US-A- 2 436 941
- US-A- 2 835 291
- US-A- 3 270 745
- US-A- 3 354 759
- US-A- 3 802 437
- US-A- 3 981 308
- US-A- 4 269 190
- US-A- 4 360 023

## Description

This invention relates to an aneurysm clip. More particularly, this invention relates to an aneurysm clip having a toggle linkage.

An aneurysm can be possessed of a thick root or neck having uneven distribution of flesh, some portions of which can be thicker or more resistant to compression than others so that to pinch the neck of the aneurysm shut to bring the walls into contact in the neck area to terminate communication between the interior of the aneurysm and the interior of the blood vessel to which it is attached, requires a substantial force.

Aneurysm clips are shown in Sugita U.S. Patent No. 4,360,023 and in Applicant's U.S. Patents Nos. 3,802,437 and 3,827,438. However, the jaws of clips of these patents, when applied to aneurysm, assume positions in which the aneurysm's resistance to compression balances the spring bias jaw closing force with the aneurysm engaging jaw faces at an acute angle, the magnitude of which is related to the magnitude of the aneurysm's size and resistance to compression.

Further, the jaws of the clips are parallel only when nothing is between the jaws to provide resistance to jaw closure, that is, when the clip is fully closed with the jaws in flatwise engagement. The opposed aneurysm engaging faces of the jaws are not parallel in aneurysm neck closing relation to an aneurysm, and, do not lock in operative aneurysm closing relation with their opposed faces parallel and at a predetermined spacing.

### BRIEF STATEMENT OF THE INVENTION

Briefly, this invention provides an aneurysm clip which includes two jaw members that are pivotally connected. One of the jaw members carries a channel shaped extension. A spring member interacts with the jaw members biasing them toward open relation. An inverted channel shaped swinging link and a rocker link form a toggle linkage between the jaw members, which linkage may be locked in predetermined aneurysm closing spaced relation and may in unlocked relation establish the limit on opening of the jaws.

### BRIEF DESCRIPTION OF THE DRAWINGS

The above and other objects and features of the invention will be apparent to those skilled in the art to which this invention pertains from the following detailed description and the drawings in which:
FIG. 1 is a view in side elevation of an aneurysm clip constructed in accordance with an embodiment of this invention, jaws thereof being in open position:
FIG. 2 is a top plan view of the clip with the jaws thereof in closed position;
FIG 3 is a view in side elevation of the clip with the jaws in closed position;
FIG. 4 is a bottom plan view of the clip with the jaws thereof in closed position; and
FIG. 5 is a fragmentary view in side elevation of a clip with the jaws thereof in closed position with fragmentary portions of sheathing sleeves in association therewith.

In the following detailed description and the drawings, like reference characters indicate like parts.

### DESCRIPTION OF PRESENTLY PREFERRED EMBODIMENT

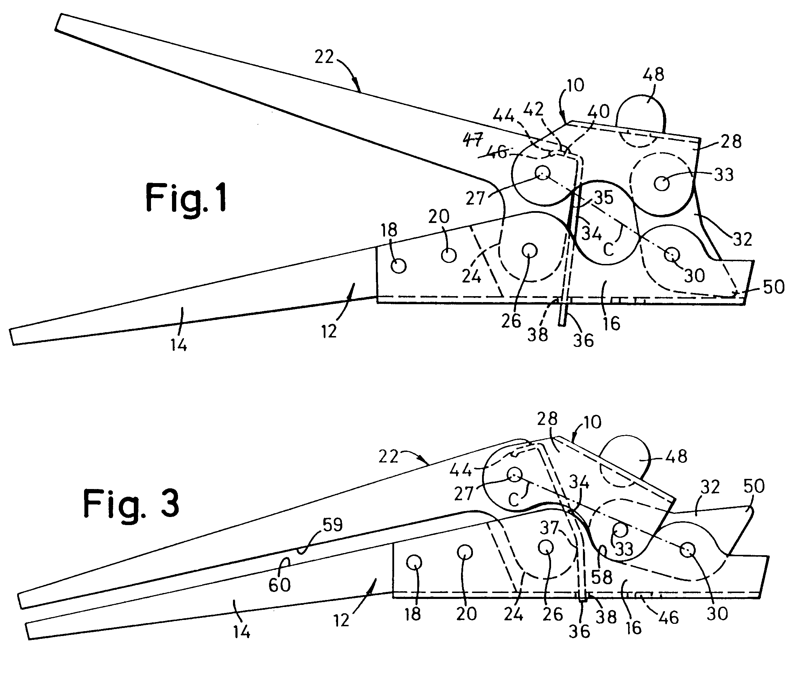

In the drawings is shown an aneurysm clip 10 which is constructed in accordance with an embodiment of this invention. The clip 10 includes a lower jaw member assembly 12 including a jaw member 14 and a channel shaped extension 16. The jaw member 14 and the channel shaped extension 16 are held in assembled relation by pins 18 and 20. An upper jaw member 22 has an angled arm 24. A pivot pin 26 pivotally connects the angled arm 24 to the channel shaped extension 16 of the lower jaw member 14. The jaw members are pivoted together to swing between an open position shown in FIG. 1 and a closed position shown in FIG. 3 in which gripping portions of the jaw members are in aneurysm gripping position. A pivot pin 27 connects the upper jaw member 22 to a swinging link 28. A pivot pin 30 connects a rocker link 32 to the channel shaped extension 16. The rocker link 32 is pivotally connected to the swinging link 28 by a pivot pin 33. The swinging link 28 and the rocker link 32 form a toggle linkage between portions of the jaw members spaced from the jaw pivot 26. A leaf spring 34 is attached to the upper jaw member 22. One end portion 36 of the leaf spring 34 is received in a slot 38 in a web of the extension 16. An opposite end portion 40 of the leaf spring 34 is bent to approximately a right angle to a main portion of the leaf spring and is received in a slot 42 in the upper jaw member 22. A hook shaped tip 44 of the leaf spring 34 fits in an inner portion 47 of the slot 42. The upper jaw member 22 can be deformed at the slot 42 by spiking or the like so that the upper jaw member 22 and the leaf spring 34 are held in assembled relation. As the jaws of the preferred embodiment are swung from FIG. 1 position to FIG. 3 position, an increasing portion of spring 34 (viz. to point 35 in FIG. 1 and to point 37 in FIG. 3) lies against angled arm 24, shortening the stressed portion of spring 34 extending across the space between angled arm 24 and slot 38 as shown in FIGS. 1 and 3.

A slot 47 is provided in the channel shaped extension 16. A knob member 48 is mounted on a web of the swinging link 28. The slot 46 and the knob member 48 can cooperate with an appropriate applicator tool for holding, opening and closing the clip 10 and for applying the clip 10 to an aneurysm.

Leaf spring 34 urges the jaw members 14 and 22 to the open position shown in FIG. 1. In the open position of FIG. 1, rocker link 32 is in the limit position of its clockwise rotation about pin 30 with its tail portion 50 engaging the web-shaped extension 16 of lower jaw 14. In said open limit position, pivot pin 33 is supported by rocker 32 in a corresponding fixed position relative to extension 16 as shown in FIG. 1 and supports swinging link 28, which in turn supports pivot pin 27 in a corresponding fixed position as well, thus by mechanical linkage establishing the open limit position of upper jaw 22 in relation to lower jaw 14.

When the aneurysm clip is closed with the jaws 14 and 22 in the relationship depicted in FIG. 3, the axes of pivot pins 33 and 26 are on the same side of the line C intersecting the axes of pins 30 and 27, that is, the knee of the toggle linkage is locked. As the spring bias force produced by spring 34 tends to open the jaws 14,22, it urges pivot pins 30 and 27 toward each other, biasing pivot pin 33 to remain on the same side of the line C through the axes of pins 30 and 27 in FIG. 1 as pivot pin 26. As shown in FIG. 3, swing link 28 may abuttingly engage lower jaw channel shaped extension 16 at 58 to mechanically establish a jaw closed toggle linkage limit position in which jaws 14 and 22 are indexed with their opposed faces 60,59 parallel and fixedly supported. Under such relationship of the parts of the clip, the jaws are mechanically secured in parallel fixed spaced relation to each other as shown in FIG. 3.

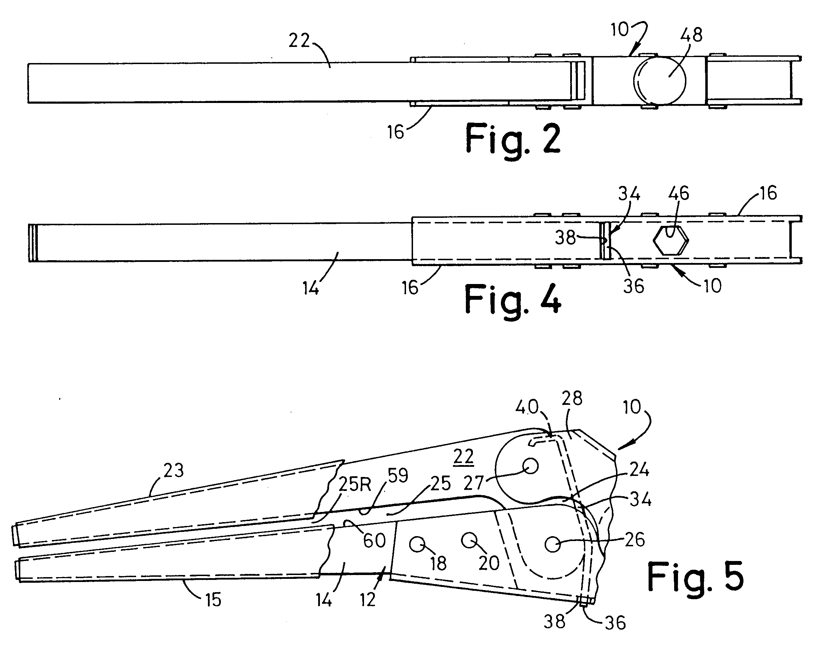

An applicator having one jaw adapted to cooperate with the spherical knob 48 and an opposed jaw having a tooth of like cross-section as and adapted to project into slot 46 can supportingly cooperate with the aneurysm clip. The slot shown in hexagonal form in FIG. 4 permits positioning of the clip in selected predetermined angular relation to the applicator having a tooth of appropriate size in hexagonal cross section. The spring bias provided by spring 34 urging the clip toward the limit position in which it is shown in FIG. 1 also urges the knob 48 and slot 46 of the clip to remain in spaced relation to each other and in cooperating relation with the jaws of an applicator, so long as the applicator jaws are retained in a spacing no greater than the maximum limit spacing of the knob and slot shown in FIG. 1 and in a spacing no less than the spacing of the knob and slot when the axis of pin 33 intersects line C. When the axis of pin 33 moves past line C toward pin 26, the knee pivot 33 is moved into a toggle locked position as shown in FIG. 3. When the parts of the clip are placed in the limit position shown in FIG. 3, opening of the applicator jaws works their detachment from the clip as the slot 46 and knob 48 remain in fixed spacing under forces flowing from spring 34 and urging pins 27 and 30 toward each other and also urging indexing stop contact between extension 16 and swinging link 28 and 58.

In the event the surgeon desires to reposition the clip, he may, either before removing the applicator from cooperating relation with the clip knob 48 in slot 46, or after re-engaging such elements with the applicator, urge the tail portion 50 of rocker link 32 from the position in which it is shown in FIG. 3 toward the position in which it is shown in FIG. 1 the short distance necessary to move pivot pin 33 from the same side of the line C through the axes of pivots 30 and 27 as pin 26 to the opposite side of said line, whereupon the spring bias urging the jaws 14 and 22 toward open position is again freed to urge such jaws, as well as slot 46 and knob 48, into wider spaced relation, which bias urges the slot and knob to remain in cooperating relation to the jaws of the applicator, thereby permitting the surgeon to adjust the position or to re-position the clip as desired and thereafter restore it to toggle locked condition. Similarly, a wedging or prying device may be inserted in the space bounded by spring 34, swinging link 28 and extension 16 in FIG. 3 and used to increase the space so that the axis of pin 33 is on the opposite side of line C from pivot 26.

As the necks or roots of aneurysms are of varying dimensions, the clips embodying the invention may be made in varying sizes, and also in any given size with respective clips having a predetermined gap 25 of one of a series of graduated sizes between the opposed jaw faces 59,60 in the closed, FIG. 3, relation so the surgeon may select a clip both as to size and as to jaw gap needed.

As the nature and physical properties of the tissue comprising the aneurysm neck or root varies, on occasion it may be desirable to provide jaws having a facing material of softer character than metal. A clip embodying the instant invention may be provided with jaw facings such as sleeves 15,23 fabricated of appropriate material of a shape which may be respectively slipped into fixed relation sheath-wise over the aneurysm gripping jaw portions 14,22 with the gap 25R between the closed sheathed jaws 14,22 being narrower than gap 25 between the unsheathed jaws depicted in FIGS. 3 and 5. Thus, if gap 25R (FIG. 5) is desired to be equal to the gap 25 of FIG. 3, the surgeon would select a clip having an interjaw gap wider than the width 25 by the sum of the thicknesses of the sleeve walls overlying the opposed jaw faces 59,60 to produce the gap reduction desired by application of respective sleeves 15,23 to the jaws of the clip. Where a narrower gap is required between bare jaws to effectively close the aneurysm neck or root, a sleeve or sleeves having thicker walls as described may be applied to one or both of the jaws, as the surgeon may elect, to effectively narrow the gap between the jaws of the clip in closed and locked condition, viz., from gap 25 width of FIGS. 3 and 5 to gap width 25R of FIG. 5, so as to more precisely adapt the jaw gap to the aneurysm root or neck to effect the desired closing of the neck of the aneurysm. Also, a series or set of jaw sheathing sleeves may be provided having different respective wall thickneses, viz., grading from thicker wall to thinner wall, than sleeves 15,23, so that a using surgeon may apply to the jaws, sleeves having walls between the opposed jaw faces which in sum equal the reduction in gap width desired to modify the gap to the size for closing the aneurysm neck. In effect, the surgeon may thus fine tune the gap in which the aneurysm neck is to be placed for closure, without sacrificing the advantage of locked parallel jaws. If further narrowing or widening of the gap is desired, sleeves of different wall thickness may be mounted on bare jaws or substituted for a sleeve or sleeves previously mounted on clip jaws.

The tendency of an aneurysm neck or root to slide toward and out of the more widely open ends of the prior art spring biased jawed clips having jaws diverging to their free, open ends, is greatly reduced, substantially eliminated, in connection with the parallel opposed jaw faces of the locked-closed clips of the instant invention.

While clips having straight parallel aneurysm neck engaging jaws are shown herein, such jaws may be curved with an even width gap between them and mountable and demountable sleeves for adjusting the gap for receiving an aneurysm neck.

The aneurysm clip is illustrated in the drawings and described above is subject to structural modification without departing from the spirit and scope of the appended claims.

Having described my invention, what I claim as new and desire to secure by letters patent is:

## Claims

1. An aneurysm clip (10) which comprises a pair of jaw members (14,22) pivoted together to swing between an open position in which gripping opposing faces of the jaw members (14,22) are spaced and an aneurysm gripping position in which the gripping opposing faces of the jaw members (14,22) are locked parallel to each other and at a predetermined spacing, a toggle linkage (28,32) between the jaw members (14,22), and spring means (34) alternately urging the toggle linkage (28,32) to the open position and to the aneurysm gripping position.

2. An aneurysm clip (10) as in claim 1 in which one link (28) of the toggle linkage (28,32) is channel shaped and portions of the other link (32) of the toggle linkage (28,32) and one of the jaw members (22) are received between flanges of the first mentioned link (28).

3. An aneurysm clip (10) as in claim 1 or claim 2 in which the gripping portion of at least one of the jaw members (14,22) carries a sleeve (23,15) having a selected thickness so that aneurysm gripping portions (14,22) of the clip (10) are at a selected gap when the clip (10) is locked in the aneurysm gripping portion.

4. An aneurysm clip (10) as in any of claims 1 to 3 in which the gripping portions of both jaw members (14,22) carry sleeves (23,15) having selected thicknesses so that aneurysm gripping portions (14,22) of the clip (10) are at a selected gap when the clip (10) is locked in the aneurysm gripping condition.

5. An aneurysm clip (10) as in any of claims 1 to 4 in which one jaw member (14) and a toggle link (28) pivoted to the other jaw member (22) have means for cooperating with an applicator and said means for cooperating with an applicator are urged to move away from each other by said spring means (34) while the latter urges the toggle linkage (28,32) toward the open position.

6. An aneurysm clip (10) as in any of claims 1 to 5 in which one link of the toggle linkage (28) engages one jaw member (14) locking the toggle (28,32) against movement beyond said position of engagement to index the gripping portions of the jaw members (14,22) in spaced predetermined aneurysm gripping position.

7. An aneurysm clip (10) as in any of claims 1 to 6 in which one link (32) of the toggle has means for engaging a jaw member (14) to limit rotation of said one link (32) about an axis of pivoting relative to said jaw member (14) to limit opening of the jaws.

8. An aneurysm clip (10) as in claim 7 in which said means on one link (32) of the toggle (28,32) for engaging a jaw member (14) may be urged toward engagement with said jaw member (14) to move the toggle linkage (28,32) from the locked position to a position from which said spring means (34) may urge the jaws (14,22) to open position.

9. An aneurysm clip (10) as in any of claims 1 to 8 in which one jaw member (14) and a toggle link (28) pivoted to the other jaw member (22) have means for cooperating with an applicator and said means for cooperating with an applicator are urged to move away from each other by said spring (34) means while the latter urges the toggle linkage (28,32) toward the open position.

## Patentansprüche

1. Aneurysma-Clip (10) mit zwei Klauengliedern (14, 22), die zusammengelenkt sind, um zwischen einer offenen Position, in der gegenüberliegende Greifflächen der Klauenglieder (14, 22) beabstandet sind, und einer Aneurysma-Greifposition zu schwenken, in der die gegenüberliegenden Greifflächen der Klauenglieder (14, 22) parallel zueinander und in einem vorgegebenen Abstand verriegelt sind, einer Kniehebel-Kopplung (28, 32) zwischen den Klauengliedern (14, 22), und mit einer Federeinrichtung (34), um die Kniehebel-Kopplung (28, 32) alternierend in die offene oder die Aneurysma-Greifposition zu drücken.

2. Aneurysma-Clip (10) nach Anspruch 1, bei dem ein Glied (28) des Kniehebel-Gelenks (28, 32) rinnenartig geformt ist und Teile des anderen Gliedes (32) der Kniehebel-Kopplung (28, 32) und eines der Klauenglieder (22) zwischen den Flanken des erstgenannten Gliedes (28) aufgenommen sind.

3. Aneurysma-Clip (10) nach Anspruch 1 oder 2, bei dem der Greifabschnitt zumindest eines der Klauenglieder (14, 22) eine Hülse (23, 15) einer bestimmten Dicke trägt, so daß sich Aneurysma-Greifabschnitte (14, 22) des Clips (10) in einem bestimmten Abstand befinden, wenn der Clip (10) in der Aneurysma-Greifposition verriegelt ist.

4. Aneurysma-Clip (10) nach einem der Ansprüche 1 bis 3, bei dem die Greifabschnitte beider Klauenglieder (14, 22) Hülsen (23, 15) bestimmter Dicke tragen, so daß sich Aneurysma-Greifabschnitte (14, 22) des Clips (10) in einem bestimmten Abstand befinden, wenn die Klammer (10) in der Aneurysma-Greifposition verriegelt ist.

5. Aneurysma-Clip (10) nach einem der Ansprüche 1 bis 4, bei dem ein Klauenglied (14) und ein Kniehebel-Glied (28), das am anderen Klauenglied (22) angelenkt ist, Einrichtungen zum Zusammenwirken mit einem Applikator aufweisen, und diese Einrichtungen zum Zusammenwirken mit einem Applikator mittels der Federeinrichtung (34) voneinander weg gedrückt werden, während die Federeinrichtung die Kniegelenk-Kopplung (28, 32) in die offene Position drückt.

6. Aneurysma-Clip (10) nach einem der Ansprüche 1 bis 5, bei dem ein Glied der Kniehebel-Kopplung (28, 32) einen Klauenglied (14) erfaßt, so daß die Kniehebel-Kopplung (28, 32) gegen Bewegung über die Erfassungsposition hinaus verriegelt ist, um die Greifabschnitte der Klauenglieder (14, 22) in einer beabstandeten vorgegebenen Aneurysma-Greifposition zu halten.

7. Aneurysma-Clip (10) nach einem der Ansprüche 1 bis 6, bei dem ein Glied (32) der Kniehebel-Kopplung (28, 32) Einrichtungen zum Erfassen eines Klauengliedes (14) aufweist, so daß die Drehung des einen Gliedes (32) um eine Schwenkachse relativ zu diesem Klauenglied (14) begrenzt ist, um die Öffnung der Klauenglieder zu begrenzen.

8. Aneurysma-Clip (10) nach Anspruch 7, wobei die Einrichtungen an einem Glied (32) der Kniehebel-Kopplung (28, 32) zum Erfassen eines Klauengliedes (14) in Richtung Erfassung des Klauengliedes (14) gedrückt werden können, um die Kniehebel-Kopplung (28, 32) aus der verriegelten Position in eine Position zu bewegen, von der aus die Federeinrichtung (34) die Klauenglieder (14, 22) in die offene Position drücken kann.

9. Aneurysma-Clip (10) nach einem der Ansprüche 1 bis 8, bei dem ein Klauenglied (14) und ein am anderen Klauenglied (22) angelenktes Kniehebelglied (28) Einrichtungen zum Zusammenwirken mit einem Applikator aufweisen, und diese Einrichtungen zum Zusammenwirken mit einem Applikator mittels der Federeinrichtung (34) voneinander weg gedrückt werden, während die Federeinrichtung die Kniegelenk-Kopplung (28, 32) zur offenen Position hin drückt.

## Revendications

1. Pince (10) pour anévrisme qui comporte une paire d'éléments (14, 22) formant mâchoire pivotant mutuellement pour basculer entre une position ouverte dans laquelle des faces opposées de saisie des éléments (14, 22) formant mâchoire sont espacées et une position de saisie d'anévrisme dans laquelle les faces opposées de saisie des éléments (14, 22) formant mâchoire sont verrouillées parallèlement l'une à l'autre et selon un écartement prédéterminé, une liaison articulée (28, 32) située entre les éléments (14, 22) formant mâchoire, et des moyens (34) formant ressort rappelant de manière alternée la liaison articulée (28, 32) vers la position ouverte et vers la position de saisie d'anévrisme.

2. Pince (10) pour anévrisme selon la revendication 1, dans laquelle une barre de liaison (28) de la liaison articulée (28, 32) a la forme d'un canal et des parties de l'autre barre de liaison (32) de la liaison articulée (28, 32) et d'un des éléments (22) formant mâchoire sont reçues entre les rebords de la première barre de liaison (28) mentionnée.

3. Pince (10) pour anévrisme selon la revendication 1 ou la revendication 2, dans laquelle la partie de saisie d'au moins un élément (14, 22) formant mâchoire supporte un manchon (23, 15) ayant une épaisseur voulue, de sorte que les parties de saisie (14, 22) d'anévrisme de la pince (10) sont situées au niveau d'un espacement voulu lorsque la pince (10) est verrouillée dans la position de saisie d'anévrisme.

4. Pince (10) pour anévrisme selon l'une quelconque des revendications 1 à 3, dans laquelle les parties de saisie des deux éléments (14, 22) formant mâchoire supportent des manchons (23, 15) ayant des épaisseurs voulues, de sorte que les parties de saisie (14, 22) d'anévrisme de la pince (10) sont situées au niveau d'un espacement voulu lorsque la pince (10) est verrouillée dans l'état de saisie d'anévrisme.

5. Pince (10) pour anévrisme selon l'une quelconque des revendications 1 à 4, dans laquelle un élément (14) formant mâchoire et une barre de liaison articulée (28) pivotant sur l'autre élément (22) formant mâchoire comportent des moyens pour coopérer avec un applicateur et lesdits moyens pour coopérer avec l'applicateur sont repoussés par lesdits moyens (34) formant ressort pour s'éloigner l'un de l'autre, pendant que ces derniers repoussent la liaison articulée (28, 32) vers la position ouverte.

6. Pince (10) pour anévrisme selon l'une quelconque des revendications 1 à 5, dans laquelle une barre de liaison (28) de la liaison articulée (28, 32) vient en contact avec un élément (14) formant mâchoire en verrouillant la liaison articulée (28, 32) à l'encontre d'un déplacement par-delà ladite position de contact pour fixer les parties de saisie des éléments (14, 22) formant mâchoire dans une position de saisie d'anévrisme ayant un espacement prédéterminé.

7. Pince (10) pour anévrisme selon l'une quelconque des revendications 1 à 6, dans laquelle une barre de liaison (32) de la liaison articulée (28, 32) comporte des moyens pour venir en contact avec un élément (14) formant mâchoire afin de limiter la rotation de ladite barre de liaison (32) autour d'un axe de pivotement par rapport audit élément (14) formant mâchoire afin de limiter l'ouverture des mâchoires.

8. Pince (10) pour anévrisme selon la revendication 7, dans laquelle lesdits moyens situés sur une barre de liaison (32) de la liaison articulée (28, 32) destinés à venir en contact avec un élément (14) formant mâchoire peuvent être repoussés vers le contact avec ledit élément (14) formant mâchoire afin de déplacer la liaison articulée (28, 32) à partir de la position verrouillée vers une position à partir de laquelle lesdits moyens (34) formant ressort peuvent repousser les mâchoires (14, 22) vers une position ouverte.

9. Pince (10) pour anévrisme selon l'une quelconque des revendications 1 à 8, dans laquelle un élément (14) formant mâchoire et une barre (28) de la liaison articulée pivotant sur l'autre élément (22) formant mâchoire comportent des moyens pour coopérer avec un applicateur et lesdits moyens pour coopérer avec l'applicateur sont repoussés par lesdits moyens (34) formant ressort pour s'éloigner l'un de l'autre pendant que ces derniers repoussent la liaison articulée (28, 32) vers la position ouverte.
